# FASCICULE DE BREVET EUROPEEN

(11) **EP 0 359 645 B1**
(45) Date de publication et mention de la délivrance du brevet: **11.01.1995**
(21) Numéro de dépôt: 89402467.8
(22) Date de dépôt: 11.09.1989
(51) Int. Cl.: C12P 7/60

(54) **Procédé de séparation de l'acide céto-2 L gulonique à partir d'un moût de fermentation**
Verfahren zur Abtrennung von Keto-2L-Gulonsäure aus Fermentationsbrühe
Process for separating keto-2L-gulonic acid from a fermentation liquor

(30) Priorité: 13.09.1988 FR 8811902
(43) Date de publication de la demande: 21.03.1990
(73) Titulaire: RHONE-POULENC SANTE, 92160 Antony (FR)
(72) Inventeur: Barthole, Jean-Pierre, F-76500 Elbeuf (FR); Filippi, Jean, F-38550 Le Péage de Roussillon (FR); Jaeger-Seddik, Aurélia, F-75014 Paris (FR); Le Fur, Isidore, F-94320 Thiais (FR); Pommier, Jean-Yves, F-75013 Paris (FR)
(74) Mandataire: Pilard, Jacques

(56) Documents cités:
- EP-A- 0 213 591
- EP-A- 0 221 707
- US-A- 2 539 472
- US-A- 3 963 574

## Description

L'acide céto-2 L gulonique, intermédiaire de synthèse de l'acide ascorbique, est généralement présent dans les moûts de fermentation de microorganismes appropriés sous forme de céto-2 L gulonate de calcium.

Pour sa transformation en acide ascorbique, il est particulièrement avantageux de pouvoir disposer de l'acide céto-2 L gulonique soit sous forme libre (2KLG-H) soit sous forme de sel de sodium (2KLG-Na) sous une forme aussi pure que possible.

Selon les demandes de brevets japonais JP 52.66684 et JP 53.62894, il est connu de préparer le céto-2 L gulonate de sodium pratiquement pur à partir d'un moût de fermentation contenant du céto-2 L gulonate de calcium. Cependant la mise en oeuvre industrielle de ces procédés ne permet pas d'obtenir directement l'acide céto-2 L gulonique nécessaire à la transformation ultérieure en acide ascorbique.

Selon les brevets européens EP 213 591 et EP 221 707, l'acide céto-2 L gulonique peut être obtenu par des procédés mettant en oeuvre des membranes échangeuses d'ions qui nécessitent la purification ultérieure du produit obtenu par des méthodes habituelles de purification telles que la cristallisation ou la chromatographie.

Le brevet américain US 2 539 472 enseigne un procédé pour l'extraction d'acides hydroxycarboxyliques en présence d'amines.

Il a maintenant été trouvé, et c'est ce qui fait l'objet de la présente invention, que l'acide céto-2 L gulonique (2KLG-H) pratiquement pur peut être obtenu avec de bons rendements à partir des moûts de fermentation contenant le sel de calcium de l'acide céto-2 L gulonique par un procédé simple et facilement reproductible.

Les milieux de fermentation conduisant au 2KLG-H contiennent généralement, à côté du sel de calcium de l'acide céto-2 L gulonique, une biomasse insoluble et des impuretés organiques ou minérales, les impuretés minérales consistant essentiellement d'anions inorganiques associés à des cations métalliques tels que les ions sodium, potassium ou magnésium.

Le procédé selon l'invention consiste essentiellement en la réalisation des étapes successives suivantes :
1) lac séparation des insolubles du moût de fermentation par ultrafiltration ou par filtration en présence d'un agent floculant et d'un adjuvant de filtration ou par centrifugation en présence d'un agent floculant,
2) la concentration du milieu obtenu par évaporation sous pression réduite ou par osmose inverse,
3) l'élimination des cations minéraux du milieu concentré par addition au milieu d'acide sulfurique en quantité pratiquement stoechiométrique pour précipiter la quasi totalité du sulfate de calcium et séparation du sulfate de calcium,
4) l'élimination des cations par la mise en contact du filtrat avec une résine polymérique échangeuse de cations en cycle acide,
5) l'élimination des ions acides forts résiduels par la mise en contact du filtrat avec une résine polymérique échangeuse d'anions ,
6) la concentration du milieu sous pression réduite,
7) la séparation de l'acide céto-2 L gulonique par cristallisation après concentration supplémentaire sous pression réduite de sa solution ou par extraction avec un solvant organique choisi parmi les hydrocarbures aliphatiques ou aromatiques éventuellement halogénés contenant en solution une amine aliphatique contenant plus de 20 atomes de carbone, puis extraction par une solution aqueuse d'acide chlorhydrique, nitrique ou sulfurique puis séparation de l'acide céto-2 L gulonique après concentration à sec de la solution aqueuse obtenue.

La mise en oeuvre du procédé peut être réalisée en continu ou en discontinu.

La biomasse et les substances insolubles qui représentent généralement de 1,5 à 3 % en poids du moût entier peuvent être séparées du moût :
- soit par centrifugation après floculation au moyen d'un agent floculant tel que, par exemple, de type polyacrylamide,
- soit par filtration sous pression réduite après floculation au moyen d'un agent floculant tel que, par exemple le polyacrylamide, et addition d'un adjuvant de filtration tel que, par exemple, la farine de bois ou la terre de diatomées,
- soit par ultrafiltration à travers des membranes minérales ou organiques appropriées telles que les membranes polyvinyldifluorées ou les membranes constituées de ZrO₂ sur matrice de carbone.

Le moût débarrassé de la biomasse et des substances insolubles peut être concentré, soit par évaporation sous pression réduite à une température inférieure à 60°C jusqu'à un volume correspondant entre 1/4 et 1/3 du volume initial, soit par osmose inverse sur une membrane polysulfonée à une température voisine de 50°C jusqu'à la moitié environ de son volume.

Le moût concentré est généralement acidifié par addition d'acide sulfurique concentré en quantité pratiquement stoechiométrique par rapport au calcium présent à une température inférieure ou égale à 40°C. Le sulfate de calcium qui précipite est séparé par filtration et est lavé à l'eau. Le filtrat qui contient des cations divers dont les principaux sont le calcium non précipité, le sodium, le potassium et le magnésium est décationisé et acidifié par passage sur une colonne de résine polymérique échangeuse de cations en cycle acide, de préférence de type sulfonique.

Il est également possible, avant la concentration, de décationiser et d'acidifier le moût par passage direct sur une résine cationique sous forme acide, de préférence de type sulfonique.

Le moût débarrassé des sédiments et des cations, éventuellement concentré, peut être débarrasse des anions en solution par passage sur une résine polymérique échangeuse d'anions, de préférence de type dialkylamino.

La solution déminéralisée est alors concentrée par évaporation sous pression réduite à une température inférieure à 60°C jusqu'à environ 70 % du volume initial. La cristallisation de l'acide céto-2 L gulouique est obtenue par une concentration supplémentaire sous pression réduite à une température voisine de 40°C jusqu'à une diminution du volume comprise entre 30 et 40 % environ suivie éventuellement d'un refroidissement de la bouillie cristalline.

Il peut être particulièrement avantageux d'effectuer la cristallisation en continu dans un cristallisoir fonctionnant par évaporation sous pression réduite à une température voisine de 40°C et muni d'un échangeur externe. Les cristaux obtenus sont séparés par filtration ou par essorage puis lavés à l'eau. L'acide céto-2 L gulonique est isolé sous forme d'un monohydrate dont la pureté est généralement voisine de 100 % et dont la molécule d'eau d'hydratation peut être éliminée par chauffage sous pression réduite.

Généralement le séchage des cristaux d'acide céto-2 L gulonique monohydraté humide est effectué par transport sous un flux d'air chaud.

Selon la présente invention, l'acide céto-2 L gulonique peut aussi être obtenu après extraction du moût concentré et déminéralisé au moyen d'un solvant organique convenable choisi parmi les hydrocarbures aliphatiques ou aromatiques éventuellement halogénés contenant en solution une amine aliphatique, de préférence secondaire, contenant plus de 20 atomes de carbone. La solution organique ainsi obtenue peut être extraite par une solution aqueuse d'un acide minéral fort choisi parmi les acides chlorhydrique, sulfurique ou nitrique dont la concentration peut être comprise entre 0,5N et 3N.

La solution aqueuse ainsi obtenue peut être concentrée à sec pour conduire à un acide céto-2 L gulonique monohydraté sous forme pulvérulente dont la pureté est généralement supérieure à 90 %.

Les exemples suivants montrent comment l'invention peut être mise en pratique.

### EXEMPLE 1 - Séparation des insolubles du moût de fermentation

On utilise un moût de fermentation obtenu par culture d'une souche de corynébactérium productrice d'acide céto-2 L gulonique contenant 1,6 % environ de biomasse et de substances insolubles et 10% de céto-2 L gulonate de calcium.

La biomasse et les substances insolubles peuvent être séparées selon l'une des méthodes suivantes :
1) On fait passer 123 kg de moût de fermentation contenant environ 2 kg de sédiments à travers une membrane d'ultrafiltration CARBOSEP® dont la porosité est de 80 000 DA, à une température de 40°C. On obtient ainsi 112 kg de perméat exempt de produits insolubles et 11 kg de rétentat contenant la totalité des sédiments et dans lequel la concentration en céto-2 L gulonate de calcium est égale à celle dans le perméat.
   Le rétentat est épuisé par diafiltration par additions successives d'eau au rétentat au cours d'une ultrafiltration identique à celle décrite précédemment. La diafiltration est arrêtée lorsque la teneur en céto-2 L gulonate de calcium dans les perméats réunis correspond à un taux de récupération d'au moins 99 %.
2) A 500 g de moût entier, agité vigoureusement, on ajoute 20 cm3 d'une solution aqueuse à 5 g/litre de floculant du type polyacrylamide FLOERGER 8850®. Après quelques minutes de contact, on ajoute, en maintenant la vigoureuse agitation, 10 g de farine de bois.
   Le mélange obtenu est filtré sur toile filtrante sous une pression réduite (300 mm de mercure ; 40 kPa).
   Le gâteau de filtration est lavé par 50 cm3 d'eau.
   La perte en céto-2 L gulonate de calcium est inférieure à 2 %.
   Le poids du filtrat contenant 9,4 % de céto-2 L gulonate de calcium est de 520 g.
3) 200 litres de moût entier contenant 2,7 % de sédiments sont floculés à l'aide de 8 litres d'une solution aqueuse à 5 g/ litre d'un agent floculant du type polyacrylamide (FLOERGER 8850®) puis sont introduits en continu au débit de 2000 litres/heure dans une clarificatrice centrifuge à assiettes de 7200 m2 de surface équivalente.

On recueille un moût clarifié contenant 0,1 % de sédiments.

### EXEMPLE 2 - Concentration du moût

Le moût débarrassé des insolubles peut être concentré selon l'une des méthodes suivantes :
1) On concentre 225 litres de moût exempt d'insolubles par évaporation sous pression réduite (72 mm de mercure ; 9,5 kPa) à une température de 47°C jusqu'à un volume de 62 litres.
   La dégradation thermique est inférieure à 0,5 %.
2) On concentre 60 litres de moût exempt d'insolubles par passage à travers un module d'osmose inverse équipé de membranes polysulfonées (PCI Z 99®) à un débit de 25 litres/h.m2 et à une température inférieure ou égale à 50°C jusqu'à obtenir un volume de 30 litres.

### EXEMPLE 3 - Précipitation du sulfate de calcium

Dans un réacteur agité, maintenu à une température inférieure ou égale à 40°C et contenant 2 litres de moût concentré obtenu dans les conditions décrites dans l'exemple 2-1, on ajoute une quantité d'acide sulfurique concentré correspondant, en moles, à la quantité totale de calcium présent dans le réacteur soit 100 cm3.

Le sulfate de calcium qui précipite sous forme de dihydrate est séparé par filtration et lavé à l'eau.

Le filtrat et les lavages réunis contiennent 99,5 %, en moles, de l'acide céto-2 L gulonique initial.

Le rendement de l'élimination du calcium est de 95 %.

### EXEMPLE 4 - Elimination des cations

On passe 3 litres du filtrat obtenu à l'exemple 3 (avant d'être mélangé aux eaux de lavage du gâteau de sulfate de calcium) sur une colonne de 80 cm de hauteur et de 5 cm de diamètre contenant 1,6 litre de résine cationique forte (Amberlite IRC 120®) en cycle acide.

Après lavage, on obtient ainsi 5,5 litres de moût dans lequel la teneur en cendres sulfuriques est inférieure à 1 % par rapport à l'acide céto-2 L gulonique présent dans la solution.

Le taux de récupération de l'acide céto-2 L gulonique est supérieur à 99,5 %.

### EXEMPLE 5 -Elimination des anions

On fait passer 0,4 litre du moût obtenu à l'exemple 4 à travers une colonne de 10 cm de hauteur et 1,4 cm de diamètre contenant 15 cm3 de résine anionique faible (Amberlite IRA 93®) afin de diminuer d'un facteur 10 la teneur en acide sulfurique dans le moût acidifié.

La perte en acide céto-2 L gulonique n'excède pas 2 % de la quantité introduite.

### EXEMPLE 6 - Cristallisation de l'acide céto-2 L gulonique

5000 g de moût obtenu dans les conditions de l'exemple 5 sont concentrés par évaporation de façon à obtenir 3430 g d'une solution contenant 1170 g d'acide céto-2 L gulonique et 255 g d'impuretés.

Le concentrat ainsi obtenu est concentré partiellement sous pression réduite (40 mm de mercure ; 5,3 kPa) à une température de 40°C jusqu'à un poids de 2010 g. Cette concentration supplémentaire conduit à la cristallisation de l'acide céto-2 L gulonique hydraté. La bouillie de cristaux est refroidie à 25°C. Les cristaux sont séparés par filtration et lavés à l'eau. On obtient ainsi 930 g d'acide céto-2 L gulonique hydraté dont la pureté est supérieure à 99 % et dont la teneur en cendres sulfuriques est inférieure à 0,1 %.

Le rendement est de 73 %.

Les eaux-mères de filtration et les eaux de lavage sont réunies puis concentrées sous pression réduite (40 mm de mercure ; 5,3 kPa) jusqu'à un poids de 710 g. Après refroidissement, les cristaux sont séparés par filtration et lavés à l'eau. On obtient ainsi 285 g d'acide céto-2 L gulonique monohydraté dont la pureté est de 89 %.

Les cristaux d'acide céto-2 L gulonique monohydraté humides (5,8 % d'eau) dont la pureté est supérieure à 99 % (9400 g) sont séchés par de l'air à 75°C circulant à 5 m/s dans un séchoir à transport pneumatique, le temps de séjour étant de 3 secondes.

On obtient ainsi 8850 g d'acide céto-2 L gulonique monohydraté. L'acide céto-2 L gulonique monohydraté peut être déshydraté par chauffage à 40°C sous pression réduite (5 mm de mercure; 0,67 kPa) pendant plusieurs heures.

### EXEMPLE 7

On met en contact 1 litre de moût débarrassé des sédiments et des cations contenant 80 g d'acide céto-2 L gulonique ayant une pureté de 84 % avec 1 litre d'une solution de 260 g d'ADOGEN 83 (Marque déposée SCHERING) dans le kérosène pendant 0,5 heure à 50°C.

L'acide céto-2 L gulonique (83 %) qui est extrait dans la phase organique est réextrait quantitativement par 690 cm3 d'une solution aqueuse d'acide nitrique 1N.

La solution concentrée à sec fournit 81,5 g d'un produit cristallisé contenant 89 % d'acide céto-2 L gulonique monohydraté.

## Revendications

1. Procédé pour la séparation de l'acide céto-2 L gulonique à partir d'un moût de fermentation contenant le sel de calcium de l'acide céto-2 L gulonique qui comprend :
1) la séparation des insolubles du moût de fermentation par ultrafiltration ou par filtration en présence d'un agent floculant et d'un adjuvant de filtration ou par centrifugation en présence d'un agent floculant,
2) la concentration du milieu obtenu par évaporation sous pression réduite ou par osmose inverse,
3) l'élimination des cations minéraux du milieu concentré par addition au milieu d'acide sulfurique en quantité pratiquement stoechiométrique pour précipiter la quasi totalité du sulfate de calcium et séparation du sulfate de calcium,
4) l'élimination des cations par la mise en contact du filtrat avec une résine polymérique échangeuse de cations en cycle acide,
5) l'élimination des ions acides forts résiduels par la mise en contact du filtrat avec une résine polymérique échangeuse d'anions ,
6) la concentration du milieu sous pression réduite,
7) la séparation de l'acide céto-2 L gulonique par cristallisation après concentration supplémentaire sous pression réduite de sa solution ou par extraction avec un solvant organique choisi parmi les hydrocarbures aliphatiques ou aromatiques éventuellement halogénés contenant en solution une amine aliphatique contenant plus de 20 atomes de carbone, puis extraction par une solution aqueuse d'acide chlorhydrique, nitrique ou sulfurique puis séparation de l'acide céto-2 L gulonique après concentration à sec de la solution aqueuse obtenue.

## Claims

1. Process for separating 2-keto-L-gulonic acid from a fermentation broth containing the calcium salt of 2-keto-L-gulonic acid which comprises:
1) separating the insolubles from the fermentation broth by ultrafiltration or by filtration in the presence of a flocculating agent and a filter aid or by centrifugation in the presence of a flocculating agent,
2) concentrating the medium obtained by evaporation under reduced pressure or by reverse osmosis,
3) removing inorganic cations from the concentrated medium by adding sulphuric acid to the medium in a practically stoichiometric quantity in order to precipitate practically all the calcium sulphate and separating the calcium sulphate,
4) removing the cations by bringing the filtrate into contact with a polymeric cation-exchange resin in acid cycle,
5) removing the residual strong acid ions by bringing the filtrate into contact with a polymeric anion-exchange resin,
6) concentrating the medium under reduced pressure,
7) separating the 2-keto-L-gulonic acid by crystallization after additional concentration under reduced pressure of its solution or by extracting with an organic solvent chosen from optionally halogenated aliphatic or aromatic hydrocarbons containing, in solution, an aliphatic amine containing more than 20 carbon atoms, then extracting with an aqueous solution of hydrochloric, nitric or sulphuric acid and then separating the 2-keto-L-gulonic acid after concentrating to dryness the aqueous solution obtained.

## Patentansprüche

1. Verfahren zur Abtrennung von Keto-2L-gulonsäure aus Fermentationsbrühe, die das Calciumsalz der Keto-2L-gulonsäure enthält, das umfaßt:
1) die Abtrennung der unlöslichen Bestandteile der Fermentationsbrühe durch Ultrafiltration oder Filtration in Gegenwart eines Flockungsmittels und eines Filtrationshilfsmittels oder durch Zentrifugation in Gegenwart eines Flockungsmittels,
2) die Konzentrierung des erhaltenen Mediums durch Evaporation unter vermindertem Druck oder durch inverse Osmose,
3) die Eliminierung der Mineralkationen des konzentrierten Mediums durch Zugabe von Schwefelsäure zum Medium in praktisch stöchiometrischer Menge, um fast die gesamte Menge an Calciumsulfat anszufallen, und die Abtrennung des Calciumsulfats,
4) die Eliminierung der Kationen dadurch, daß das Filtrat mit einem polymeren Kationenaustauscherharz in saurem Zyklus in Kontakt gebracht wird,
5) die Eliminierung der restlichen starken Säureionen dadurch, daß das Filtrat mit einem polymeren Anionaustauscherharz in Kontakt gebracht wird,
6) die Konzentrierung des Milieus unter vermindertem Druck,
7) die Abtrennung der Keto-2L-gulonsäure durch Kristallisation nach zusätzlicher Konzentrierung ihrer Lösung unter vermindertem Druck oder durch Extraktion mit einem organischen Lösungsmittel, ausgewählt aus den aliphatischen oder aromatischen gegebenenfalls halogenierten Kohlenwasserstoffen, das ein aliphatisches Amin mit mehr als 20 Kohlenstoffatomen gelöst enthält, dann Extraktion mit einer wäßrigen Salzsäure-Salpetersäure oder Schwefelsäurelösung, dann Abtrennung der Keto-2L-gulonsäure nach Einengen der erhaltenen wäßrigen Lösung bis zur Trockne.
